# EUROPEAN PATENT SPECIFICATION

(11) **EP 1 574 186 B1**
(45) Date of publication and mention of the grant of the patent: **04.06.2008**
(21) Application number: 05251376.9
(22) Date of filing: 08.03.2005
(51) Int. Cl.: A61F 2/46, A61B 19/00

(54) **Navigated stemmed orthopaedic implant inserter**
Navigierte Vorrichtung zum Einsetzen eines Schaftkomponents für orthopädische Implantaten
Dispositif d'insertion navigué pour implants orthopédiques à tiges

(30) Priority: 08.03.2004 US 795837
(43) Date of publication of application: 14.09.2005
(73) Proprietor: Zimmer Technology, Inc., Warsaw, IN 46580 (US)
(72) Inventor: Grimm, James E., Winona Lake, Indiana 46590 (US); Rangaiah, Chetan, Warsaw, Indiana 46581 (US); McGinley, Shawn E., Fort Wayne, Indiana 46814 (US); Walriven, Dale E., Warsaw, Indiana 46580 (US)
(74) Representative: Mays, Julie

(56) References cited:
- EP-A- 0 207 873
- EP-A- 0 940 127
- EP-A- 0 940 128
- DE-U1- 20 021 494
- US-A1- 2003 220 689

## Description

### FIELD OF THE INVENTION

The present invention relates to inserter instruments for orthopaedic implants to which the closest prior art is EP-A-0 207 873. This document discloses a stemmed implant inserter comprising:
a stem engaging member engageable with the stemmed orthopaedic implant in rigid relative arrangement and including a ring-shaped head having a bore with a bore axis for receiving a neck of the stemmed orthopaedic implant;
a stem locking member mounted to the inserter for translation along a stem locking axis transverse to the bore axis of the head, the stem locking member being movable between a first position in which the neck of the stemmed orthopaedic implant may be disengaged from the head and a second position in which the neck is prevented from being disengaged from the head; and
an actuator being able to convert rotary input to the actuator into linear motion to move the stem locking member from the first position to the second position.
In particular, the present invention relates to a navigated inserter for placing a stemmed orthopaedic implant into an intramedullary canal of a bone.

### BACKGROUND

Many surgical procedures are now performed with surgical navigation systems in which sensors detect tracking elements attached in known relationship to objects in the surgical suite such as a surgical instrument, implant, or patient body part. The sensor information is fed to a computer that then triangulates the three dimensional position of the tracking elements within the surgical navigation system coordinate system. Thus, the computer can resolve the position and orientation of the objects and display the position and orientation for surgeon guidance. For example, the position and orientation of an instrument or implant can be shown superimposed on an image of the patient's anatomy obtained via X-ray, CT scan, ultrasound, or other technology.

European patent application publication no. 0 940 127 in the name of Benoist Girard SAS discloses a non-navigated prosthesis inserter having a trigger for operating the stem locking member. However, this trigger only allows a minimal degree of control of the stem locking member during translation between the locking and non-locking positions.

US patent application publication no. US 2003/0220689 in the name of Ritland et al discloses an implant guide having navigation means. However, there is no mention of engaging an implant with a stem locking member prior to insertion, or of any means by which the movement of such a member between the locking and non-locking positions could be controlled.

### SUMMARY

The present invention provides a navigated stemmed orthopaedic implant inserter as defined in the attached set of claims.

### BRIEF DESCRIPTION OF THE DRAWINGS

Various examples of the present invention will be discussed with reference to the appended drawings. These drawings depict only illustrative examples of the invention and are not to be considered limiting of its scope.
FIG. 1 is an exploded perspective view of an illustrative inserter according to the present invention;
FIG. 2 is a perspective view of the inserter of FIG. 1; and
FIG. 3 is a cross sectional view of the inserter of FIG. 1.

### DESCRIPTION OF THE ILLUSTRATED EXAMPLES

Stemmed orthopaedic implants are typically, and successfully, positioned within an intramedullary canal of a bone by inserting the stemmed orthopaedic implant into the bone until it abuts known anatomic land marks. For example, a hip stem implant 100 may be inserted until it abuts the wall of the prepared intramedullary canal of a femur and/or until a collar on the implant abuts the edge of the resected femur. The hip stem implant 100 may be rotated to visually align a neck 104 or other feature. For example, the hip stem implant 100 may be rotated to align a neck 104 with the position that the anatomic femoral neck once occupied. Other stemmed orthopaedic implants, including humeral implants in shoulder replacement surgery, are inserted similarly. The present investigators have determined that stemmed orthopaedic implants may be advantageously inserted using surgical navigation technology to track the position of the implant relative to the bone.

To permit tracking of the stemmed orthopaedic implant 100, a navigated stemmed implant inserter 150 is provide that includes a stem engaging member 200 and a reference member 550 trackable by the surgical navigation system. The stem engaging member 200 locks onto the stemmed orthopaedic implant 100 in a rigid manner so that the reference member 550 is supported in fixed relationship to the stemmed orthopaedic implant 100. The relationship of the stemmed orthopaedic implant 100 to the reference member 550 is registered in the surgical navigation system after the inserter 150 is attached to the stemmed orthopaedic implant 100. Registration may be accomplished, for example, by engaging the stemmed orthopaedic implant 100 with a calibration block having a known geometry and being trackable within the surgical navigation system and activating the surgical navigation system to determine the relationship between the calibration block and reference member 550. The surgical navigation system may then resolve and record the relationship of the stemmed orthopaedic implant 100 to the reference member 550 such that the relationship is known to the system. Once the inserter 150 is attached to the stemmed orthopaedic implant 100 and the relationship is registered in the surgical navigation system, the relationship is held rigid so that it does not change during the course of inserting the stemmed orthopaedic implant 100 into a bone. If the manufacturing tolerances of the inserter 150 and stemmed orthopaedic implant 100 are held within a sufficiently narrow range such that the inserter 150 attaches in a repeatable manner, the relationship of the stemmed orthopaedic implant 100 to the reference member 550 may be predetermined and recorded within the system such that relationship need not be calibrated each time the inserter is attached 150. The stem engaging member 200 may attach to any portion of the stemmed orthopaedic implant 100 that will not interfere with insertion of the stemmed orthopaedic implant 100 into the bone. For example, the stem engaging member 200 may attach to a neck 104, proximal body 106, extraction hole 108, or other suitable feature.

The reference member 550 is trackable by a surgical navigation system that may include multiple sensors at known locations that feed reference member position information to a computer. The computer may then triangulate the three dimensional position of the reference member 550 within the surgical navigation coordinate system. The surgical navigation system may determine the position and orientation of the stemmed orthopaedic implant 100 by detecting the position and orientation of the reference member 550 and resolving the position and orientation of the stemmed orthopaedic implant 100 from the known relationship between the reference member 550 and the stemmed orthopaedic implant 100.

The reference member 550 may be detectable electromagnetically, acoustically, by imaging, or by other suitable detection means. Furthermore, the reference member 550 may be active or passive. Examples of active reference members 550 may include electromagnetic field emitters in an electromagnetic system, members that generate an electrical current when placed in an electromagnetic field in an electromagnetic system, light emitting diodes in an imaging system, and ultrasonic emitters in an acoustic system, among others. Examples of passive tracking elements may include elements with reflective surfaces. For example, reflective spheres or discs may be attached to the orthopaedic guide and detected by an imaging system.

The navigated stemmed implant inserter 150 is useful to position a stemmed orthopaedic implant 100 in a desired position within a bone. For example, the depth that the stemmed orthopaedic implant 100 is inserted into the bone, the rotation of the stemmed orthopaedic implant 100 about the axis of the intramedullary canal of the bone, the anterior-posterior tilt of the stemmed orthopaedic implant 100 relative to the bone, the medial-lateral tilt of the stemmed orthopaedic implant 100 relative to the bone, and/or other position parameters of the stemmed orthopaedic implant 100 within the bone may have desired values. By using the navigated stemmed implant inserter 150 of the present invention, these parameters can be measured and adjusted. In addition, the bone may be prepared by broaching an opening in the bone with a rasp that also connects to a reference member 550. By recording the final position of the rasp, the stemmed orthopaedic implant 100 may be placed in the same position as the rasp using the navigated stemmed implant inserter 150. This is particularly beneficial where the stemmed orthopaedic implant 100 is being press fit into the broached opening. By matching the rasp orientation, the surgeon can minimize the likelihood of splitting the femur. The navigated stemmed implant inserter 150 may be used to insert stemmed orthopaedic implants 100 that are fixed by cementing, press fitting, polished taper sliding fit, and/or other fixation methods.

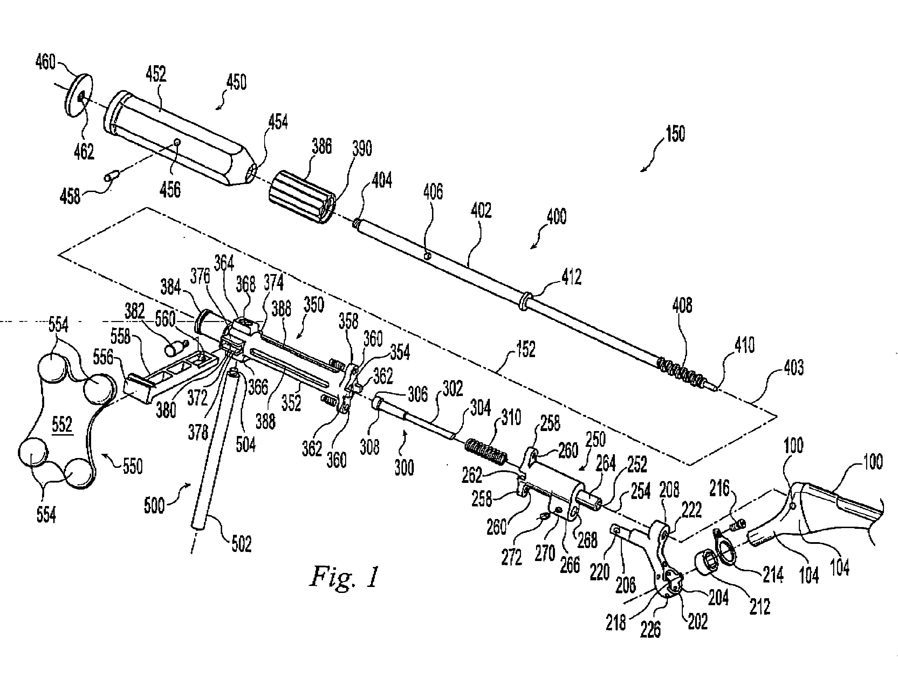

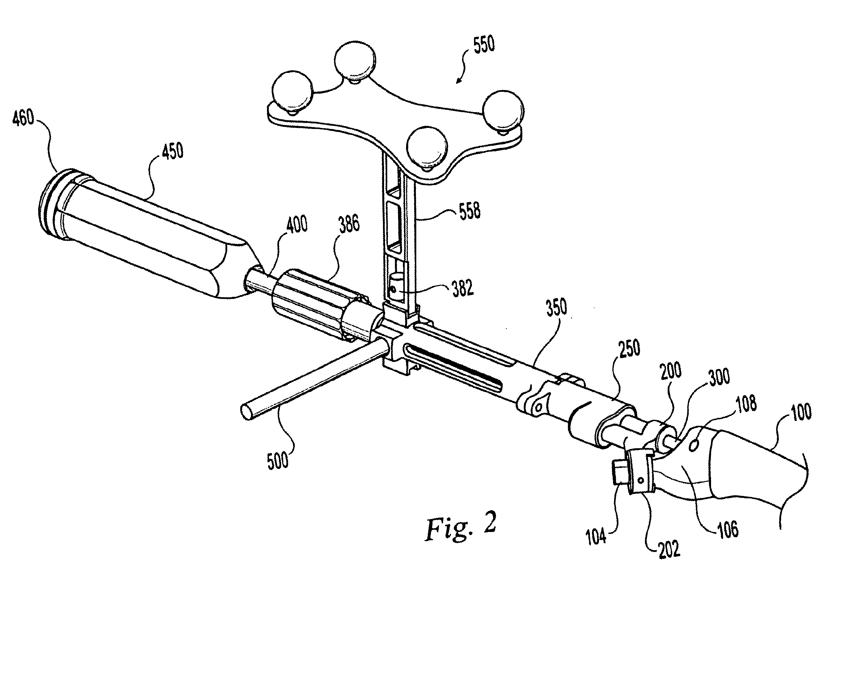

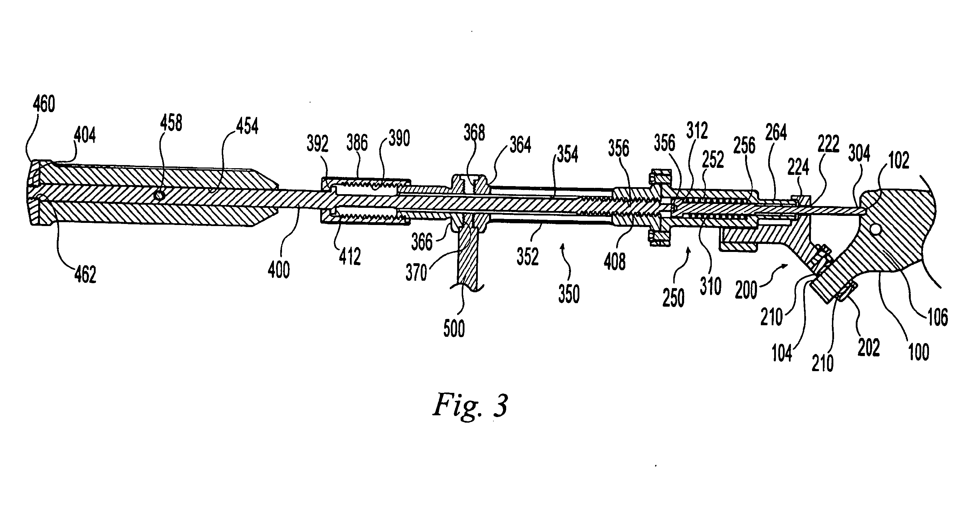

An illustrative navigated stemmed implant inserter 150 is shown in FIGS. 1-3 for use with a femoral hip stem implant 100. The following description provides a detailed explanation of this particular illustrative example. However, this detailed description should not be taken as limiting of the scope of the invention. The illustrative navigated stemmed implant inserter 150 for a femoral hip stem implant 100 includes a stem engaging member 200 for engaging a portion of the hip stem implant 100, a distal housing 250, a stem locking member 300 for locking the hip stem implant 100 in engagement with the stem engaging member 200, a proximal housing 350, an actuator 400 for activating the stem locking member 300, a primary handle 450, an auxiliary handle 500, and a reference member 550 for permitting the inserter 150 to be tracked by a surgical navigation system. In describing the inserter 150 components, the term proximal will be used to refer to relative positions nearer the primary handle 450 and the term distal will be used to refer to relative positions further from the primary handle 450.

The stem engaging member 200 includes a ring-shaped head 202 at its distal end having a through bore 204, a connector shaft 206 at its proximal end for engaging the distal housing 250, and a boss 208 intermediate the proximal and distal ends for engaging the distal housing 250. The bore 204 in the head 202 includes a lip 210. A protective sleeve 212 lines the bore 204 and abuts the lip 210. The protective sleeve 212 may be made of a material that will not mar the hip stem implant 100. For example, the protective sleeve 212 may be made of a polymer such as polyetheretherketone, polyethylene, polyester, and/or other suitable materials. A retention plate 214 fits over the sleeve 212 to retain the sleeve 212 in the bore 204. The retention plate 214 is held in place with a screw 216 inserted through the plate 214 and into the head 202. The retention plate 214 is constrained from pivoting around the screw 216 by a pair of opposed ears 218 extending distally from the head 202. The connector shaft 206 includes a dimple 220 for receiving a set screw. The boss 208 includes a through bore 222 having a non-circular cross section for engaging the stem locking member 300. The through bore 222 includes a proximal counter bore 224 for receiving a portion of the distal housing 250. The stem engaging member 200 includes transverse holes 226 through the ring-shaped head 202 to facilitate cleaning. The holes 226 permit cleaning fluid to enter between the head 202 and the protective sleeve 212. The illustrative stem engaging member 200 is particularly suited for gripping existing hip stem implants 100 that have a neck 104 engageable by the head 202. The illustrative stem engaging member 200 provides a rigid connection to permit navigated insertion of stems that were not designed with navigated insertion in mind. Thus, the illustrative stem engaging member 200 may adapt older hip stem implant 100 designs to navigation.

The distal housing 250 includes an axial through bore 252 having a longitudinal axis 254. The longitudinal axis 254 of the bore 252 is coincident with an inserter longitudinal axis 152. The bore 252 has a first, smaller, diameter near its distal end and a second, larger diameter, near its proximal end such that there is a step, or shoulder 256, formed between the proximal and distal ends. The distal housing 250 includes a radially outwardly extending flange 258 near its proximal end including threaded bores 260 for coupling the distal housing 250 to the proximal housing 350. The distal housing 250 further includes a pair of grooves 262 at its proximal end to aid in aligning the housings 250, 350 during assembly. The distal housing 250 is reduced in size at its distal end to form a nose 264 that engages the counter bore 224 of the boss 208 on the stem engaging member 200 to rotationally constrain the housings 250, 350 relative to one another. The distal housing 250 includes a radially extending boss 266 having a socket 268 the receives the connector shaft 206 of the stem engaging member 200 to couple the stem engaging member 200 to the distal housing 250. A transverse threaded bore 270 communicates with the socket 268 and receives a set screw 272 for securing the connector shaft 206 in the socket 268. The set screw aligns with the dimple 220 to positively lock the shaft 206 in the socket 268 and axially and rotationally constrain the housings 250, 350 relative to one another. With the stem engaging member 200 connected to the distal housing 250, the bores 252, 222 in the two components align to form a continuous bore.

The stem locking member 300 includes an elongated body 302 having a distal end 304 for engaging the femoral hip stem implant 100, a proximal end 306 for engaging the actuator 400, and a radially enlarged head 308 formed adjacent the proximal end 306. The distal end 304 may press against a portion of the hip stem implant 100 such as the proximal body 106. Alternatively, the distal end 304 may fit within a recess 102 in hip stem implant 100. The distal end may be rectangular, round, elliptical, or any other suitable cross sectional shape. However, in the illustrative stem locking member 300, the distal end 304 has a non-circular elliptical cross section to fit an elliptical dimple 102 existing in some femoral hip stem implants 100. For example, the VerSys^{®} Fiber Metal Taper hip stem manufactured by Zimmer, Inc. of Warsaw, Indiana includes such a dimple 102. It is advantageous for the distal end 304 to fit closely within the dimple 102 to better constrain the position of the hip stem implant 100 relative to the inserter 150.

The head 308 includes an axially aligned dimple 312 for receiving the actuator 400. A spring 310 is receive over the elongated body 302 and abuts the head 308. The stem locking member 300 is received in the axial bore 252 of the distal housing 250 with its distal end 304 extending through the axial bore 252 of the distal housing and the non-circular bore 222 of the boss 208 on the stem engaging member 200. The engagement of the non-circular distal end 304 of the stem locking member with the non-circular bore 222 ensures that the distal end 304 will be rotationally aligned with the dimple 102 in the hip stem implant 100. The spring 310 is trapped between the head 308 of the stem locking member 300 and the shoulder 256 in the distal housing 250 and biases the stem locking member 300 proximally away from the stem engaging member 200.

The illustrative inserter 150 is shown with the actuator 400 and stem locking member 300 being separate parts. This facilitates the alignment of a non-circular distal end 304 of the stem locking member 300 with a non-circular dimple on the illustrative hip stem implant 100. It also permits the actuator 400 to have a rotary action while the stem locking member 300 is constrained against rotation. However, in the case where the distal end 304 of the stem locking member 300 is circular or where it may otherwise be permitted to rotate relative to the distal housing 250, the actuator 400 and stem locking member 300 may be provided as a single piece and the spring 310 may be omitted.

The proximal housing 350 includes an elongated tubular body 352 having an axial through bore 354. Threads 356 are formed in the bore 354 adjacent the distal end to engage the actuator 400. The proximal housing 350 includes a radially outwardly extending flange 358 near its distal end including bores 360 for coupling the proximal housing 350 to the distal housing 250. The proximal housing 350 further includes a pair of tabs 362 at its distal end that engage the grooves 262 of the distal housing to aid in aligning the housings 250, 350 during assembly. With the tabs 362 inserted into the grooves 262, screws are inserted through the bores 360 in the proximal housing 350 and threaded into the bores 260 in the distal housing to couple the housings 250, 350 together with their bores 354, 252 collinearly aligned. The portion of the axial bore 354 near the distal end of the proximal housing 350 is smaller than the head 308 of the stem locking member 300 so that the stem locking member 300 is trapped between the housings 250, 350. The proximal housing 350 includes opposed auxiliary handle 500 mounting fittings 364, 366 including threaded bores 368, 370. The handle fittings 364, 366 permit the auxiliary handle 500 to be mounted in different orientations based on surgeon preference and/or to accommodate insertion of femoral stem implants 100 in both right and left femurs. The proximal housing 350 includes opposed reference member 550 mounting fittings 372, 374 including spaced apart upraised sidewalls 376 defining sockets 378. Threaded bores 380 are disposed in the bottom of the sockets 378 to receive an attachment screw 382. The reference member fittings 372, 374 permit the reference member 550 to be mounted in different orientations based on surgeon preference and/or to accommodate insertion of femoral stem implants 100 in both right and left femurs. The proximal housing 350 includes threads 384 at its proximal end for engaging a locking ring 386. The proximal housing 350 includes slots 388 to facilitate cleaning of its interior.

The actuator 400 includes an elongated shaft 402 having an axis 403 coincident with the inserter axis 152. The shaft includes threads 404 and a transverse bore 406 near its proximal end for connecting the shaft 402 to the primary handle 450. The shaft 402 includes threads 408 near its distal end for engaging the threads 356 near the distal end of the proximal housing 350. These threads 408, 356 convert rotary inputs to the actuator 400 into linear translation along the inserter axis 152. A distal tip 410 engages the dimple 312 in the head 308 of the stem locking member 300 to drive the stem locking member distally in the distal housing 250. The shaft includes a radially extending flange 412 for abutting the locking ring 386. The actuator 400 is assembled into the inserter 150 by inserting the distal tip 410 along the axial bore 354 of the proximal housing 350 until the distal actuator threads 408 abut the threads 356 of the proximal housing 350. The actuator 400 is then rotated to engage the threads 408, 356 and move the actuator distally until the distal tip 410 engages the dimple 312 in the head 308 of the stem locking member 300. The locking ring 386 is then threaded onto the proximal housing 350 by engaging internal locking ring threads 390 with the proximal housing threads 384. The locking ring 386 is advanced until a locking ring shoulder 392 engages the actuator flange 412.

The primary handle 450 includes an exterior gripping surface 452 and an axial through bore 454. A transverse bore 456 communicates from the exterior gripping surface 452 to the axial bore 454. The handle 450 is assembled onto the actuator shaft 402 by sliding the axial bore 454 over the proximal end of the shaft 402 until the transverse bore 456 in the handle 450 aligns with the transverse bore 406 in the shaft 402. A pin 458 is then inserted into the bores 456, 406 to lock the handle 450 on the shaft 402. A strike plate 460 having a threaded bore 462 is then threaded onto the threads 404 on the proximal end of the actuator shaft 402 until the strike plate 460 abuts the proximal end of the primary handle 450. The handle 450 may be used to grip the inserter 150 and manipulate it relative to the surgical site. The handle 450 may also be rotated to move the actuator 400 relative to the proximal housing 350 to activate the stem locking member 300.

The auxiliary handle 500 includes an elongated shaft 502 having threads 504 at one end for engaging the threaded bore 368, 370 in the auxiliary handle mounting fittings 364, 366 of the proximal housing 350. The auxiliary handle 500 provides additional gripping options to the surgeon and permits a counter rotation force to be applied to the proximal housing 350 while the primary handle 450 is rotated to drive the actuator 400.

The reference member 550 includes a reference member body 552 supporting reference elements 554. The reference member 550 includes a connecting portion (not shown) including a female dovetail opening for connecting to a male dovetail 556 on a reference member tower 558. The reference member tower 558 includes a through bore 560 at one end. The reference member tower 558 engages the socket 378 of the reference member mounting fittings 372, 374. With tower 558 engaged with the socket 378, the through bore 560 aligns with the threaded bore 380 so that the attachment screw 382 may be inserted through the through bore 560 and engaged with the threaded bore 380 to lock the tower in place.

In use, the neck 104 of the hip stem implant 100 is inserted into the protective sleeve 212 in the head 202 of the stem engaging member 200 until the neck 104 is pressed tightly against the sleeve 212. The primary handle 450 may then be rotated to turn the actuator 400 and cause it to translate distally as the actuator threads 408 engage the internal proximal housing threads 356. By gripping the auxiliary handle 500, a counter torque may be applied to the proximal housing 350 to facilitate turning the primary handle 450 and actuator 400 relative to the proximal housing 350. As the actuator 400 translates distally, the distal tip 410 of the actuator 400 presses against the stem locking member 300 and causes it to move distally. The stem locking member 300 compresses the spring 310 and the distal end 304 of the stem locking member 300 moves into engagement with the hip stem implant 100. In the illustrative embodiment, the distal end 304 of the stem locking member 300 engages the dimple 102 in the proximal body 106 of the hip stem implant 100. Further tightening of the primary handle 450 causes the stem locking member 300 to press tightly against the hip stem implant 100 and lock it in position relative to the stem engaging member 200. Since the bore 204 in the head 202 of the stem engaging member 200 is angled relative to the axis 254 along which the stem locking member 300 travels, the hip stem implant 100 must move transversely to that axis 254 in order for it to be withdrawn from the stem engaging member 200. With the stem locking member 300 pressed against the hip stem implant 100, the hip stem implant 100 is prevented from moving transversely and therefore it is locked in position. Alternatively, the stem locking member 300 may press against the hip stem implant 100 to create a bending moment at the neck 104 that causes the head 202 to grip the neck 104 in a wedging grip. The locking ring 386 may then be tightened against the flange 412 to provide axial and frictional rotational resistance to the actuator 400 loosening in use.

The position of the hip stem implant 100 may now be tracked by the surgical navigation system which tracks the reference elements 554 and resolves the position of the hip stem implant 100 from the known relationship between the reference elements and the hip stem implant 100. The hip stem implant 100 may be guided to a desired position and orientation in the femur such as to a desired depth, rotation, anterior-posterior tilt, medial-lateral tilt, and/or other position parameters of the hip stem implant 100 within the femur. By using the navigated stemmed implant inserter 150 of the present invention, these parameters can be measured and adjusted. In addition, the femur may be prepared by broaching an opening in the femur with a rasp that also connects to a reference member 550. By recording the final position of the rasp, the hip stem implant may be placed in the same position as the rasp using the navigated stemmed implant inserter 150. This is particularly beneficial where the hip stem implant 100 is being press fit into the broached opening. By matching the rasp orientation, the surgeon can minimize the likelihood of splitting the femur. The auxiliary handle 500 may be used to rotate the hip stem implant 100 to the desired position within the femur and the strike plate 460 may be impacted to drive the hip stem implant 100 into the femur.

The inserter 150 is disengaged from the hip stem implant 100 by loosening the locking ring 386 and then rotating the primary handle 450 to translate the actuator 400 proximally in the proximal housing 350. The spring 310 retracts the stem locking member 300 proximally to disengage it from the hip stem implant 100. The head 202 of the stem engaging member 200 may then be slipped off of the neck 104.

Although an example of a navigated stemmed implant inserter and its use have been described and illustrated in detail, it is to be understood that the same is intended by way of illustration and example only and is not to be taken by way of limitation. Accordingly, variations in and modifications to the inserter and its use will be apparent to those of ordinary skill in the art, and the following claims are intended to cover all such modifications and equivalents.

## Claims

1. A navigated stemmed implant inserter (150) for use with a stemmed orthopaedic implant(100) and a surgical navigation system during joint replacement surgery on a bone, the inserter (150) comprising:
a stem engaging member (200) engageable with the stemmed orthopaedic implant (100) in rigid relative arrangement and including a ring-shaped head (202) having a bore (204) with a bore axis for receiving a neck (104) of the stemmed orthopaedic implant (100);
a reference member (550) trackable by the surgical navigation system, the reference member (550) being supported in a known rigid relationship to the stemmed orthopaedic implant (100) such that the surgical navigation system may determine the position and orientation of the stemmed orthopaedic implant (100) relative to the bone by detecting the position and orientation of the reference member (550) and resolving the position and orientation of the stemmed orthopaedic implant (100) from a known relationship between the reference member (550) and the stemmed orthopaedic implant (100);
a stem locking member (300) mounted to the inserter for translation along a stem locking axis (254) transverse to the bore axis of the head (202), the stem locking member (300) being movable between a first position in which the neck (104) of the stemmed orthopaedic implant (100) may be disengaged from the head (202) and a second position in which the neck (104) is prevented from being disengaged from the head (202); and
an actuator (400) mounted to the inserter (150) for rotation and translation along the stem locking axis (254), the actuator (400) being able to convert rotary input to the actuator (400) into linear motion to move the stem locking member (300) from the first position to the second position, wherein the actuator (400) includes a locking ring abutment portion (412) and the stemmed implant inserter (150) further comprises a locking ring (386) threaded onto the inserter (150), the locking ring (386) being moveable between a first position in which it is spaced from the locking ring abutment portion (412) and a second position in which it abuts the locking ring abutment portion (412).

2. The navigated stemmed implant (150) inserter of claim 1 **characterised in that** the reference member (550) is detectable using a detection technology selected from the group consisting of electromagnetic, acoustical, and imaging.

3. The navigated stemmed implant inserter (150) of claim 2 **characterised in that** the reference member (550) actively produces a signal detectable by the surgical navigation system.

4. The navigated stemmed implant inserter (150) of claim 2 **characterised in that** the reference member (550) is passively detected by the surgical navigation system.

5. The navigated stemmed implant inserter (150) of any one of the preceding claims **characterised in that** the surgical navigation system is able to resolve and display one or more position parameters of the stemmed orthopaedic implant (100) relative to the bone selected from the list consisting of depth, rotation, anterior-posterior tilt, and medial-lateral tilt.

6. The navigated stemmed implant inserter (150) of any one of the preceding claims **characterised in that** the head (202) includes a sleeve (212) disposed in the bore (204) to isolate the stemmed orthopaedic implant (100) from the head (202).

7. The navigated stemmed implant inserter (150) of claim 6 **characterised in that** the sleeve (212) comprises a polymer.

8. The navigated stemmed implant inserter (150) of claim 6 or claim 7 further comprising a retention plate (214) disposed over the sleeve (212) to retain the sleeve (212) in the bore (204).

9. The navigated stemmed implant inserter (150) of any one of the preceding claims **characterised in that** the stem locking member (300) and actuator (400) are separate elongated members coaxially aligned with one another, the inserter (1.50) further comprising a spring (310) mounted adjacent the stem locking member (300) to bias the stem locking member (300) toward the actuator (400).

10. The navigated stemmed implant inserter (150) of any one of the preceeding claims **characterised in that** the locking ring abutment portion (412) comprises a radially extending flange.

11. The navigated stemmed implant inserter (150) of any one of the preceding claims further comprising an auxiliary handle (500) mounted transversely to the stem locking axis (254).

12. The navigated stemmed implant inserter (150) of claim 11 further comprising a plurality of handle mounting fittings (364, 366) such that the handle (500) may be mounted in a plurality of positions transverse to the actuator axis.

13. The navigated stemmed implant inserter (150) of any one of the preceding claims **characterised in that** the inserter (150) includes a plurality of reference member mounting fittings (372, 374) such that the reference member (550) may be mounted in a plurality of positions.

## Patentansprüche

1. Navigiertes Schaftimplantat-Setzinstrument (150) zur Verwendung mit einem orthopädischen Schaftimplantat (100) und einem chirurgischen Navigationssystem während einer Gelenkersatzoperation an einem Knochen, wobei das Setzinstrument (150) aufweist:
ein Schafteingriffselement (200), das mit dem orthopädischen Schaftimplantat (100) in einer starren relativen Anordnung in Eingriff bringbar ist und einen ringförmigen Kopf (202) aufweist, der eine Bohrung (204) mit einer Bohrungsachse zur Aufnahme eines Halses (104) des orthopädischen Schaftimplantats (100) aufweist;
ein Bezugselement (550), das durch das chirurgische Navigationssystem verfolgbar ist, wobei das Bezugselement (550) in einer bekannten starren Beziehung zum orthopädischen Schaftimplantat (100) gehalten wird, so daß das chirurgische Navigationssystem die Position und Orientierung des orthopädischen Schaftimplantats (100) relativ zum Knochen bestimmen kann, indem es die Position und Orientierung des Bezugselements (550) erfaßt und die Position und Orientierung des orthopädischen Schaftimplantats (100) aus einer bekannten Beziehung zwischen dem Bezugselement (550) und dem orthopädischen Schaftimplantat (100) auflöst;
ein Schaftverriegelungselement (300), das zur Translation längs einer Schaftverriegelungsachse (254) quer zur Bohrungsachse des Kopfes (202) am Setzinstrument angebracht ist, wobei das Schaftverriegelungselement (300) zwischen einer ersten Position, in der der Hals (104) des orthopädischen Schaftimplantats (100) vom Kopf (202) gelöst werden kann, und einer zweiten Position beweglich ist, in der verhindert wird, daß der Hals (104) vom Kopf (202) gelöst wird; und
ein Betätigungselement (400), das am Setzinstrument (150) zur Rotation und Translation längs der Schaftverriegelungsachse (254) angebracht ist, wobei das Betätigungselement (400) ein Dreheingang in das Betätigungselement (400) in eine Linearbewegung umwandeln kann, um das Schaftverriegelungselement (300) von der ersten Position zur zweiten Position zu bewegen, wobei das Betätigungselement (400) einen Verriegelungsringanschlagabschnitt (412) aufweist und das Schaftimplantat-Setzinstrument (150) ferner einen Verriegelungsring (386) aufweist, der auf das Setzinstrument (150) geschraubt ist, wobei der Verriegelungsring (386) zwischen einer ersten Position, in
der er vom Verriegelungsringanschlagabschnitt (412) beabstandet ist, und einer zweiten Position beweglich ist, in der er an den Verriegelungsringanschlagabschnitt (412) anstößt.

2. Navigiertes Schaftimplantat-Setzinstrument (150) nach Anspruch 1, **dadurch gekennzeichnet, daß** das Bezugselement (550) unter Verwendung einer Erfassungstechnik erfaßbar ist, die aus der Gruppe ausgewählt ist, die aus elektromagnetisch, akustisch und Abbildung besteht.

3. Navigiertes Schaftimplantat-Setzinstrument (150) nach Anspruch 2, **dadurch gekennzeichnet, daß** das Bezugselement (550) aktiv ein Signal erzeugt, das durch das chirurgische Navigationssystem erfaßbar ist.

4. Navigiertes Schaftimplantat-Setzinstrument (150) nach Anspruch 2, **dadurch gekennzeichnet, daß** das Bezugselement (550) durch das chirurgische Navigationssystem passiv erfaßt wird.

5. Navigiertes Schaftimplantat-Setzinstrument (150) nach einem der vorhergehenden Ansprüche, **dadurch gekennzeichnet, daß** das chirurgische Navigationssystem imstande ist, einen oder mehrere Positionsparameter des orthopädischen Schaftimplantats (100) relativ zum Knochen aufzulösen und anzuzeigen, die aus der Liste ausgewählt sind, die aus Tiefe, Rotation, anteriorer-posteriorer Neigung und medialer-lateraler Neigung besteht.

6. Navigiertes Schaftimplantat-Setzinstrument (150) nach einem der vorhergehenden Ansprüche, **dadurch gekennzeichnet, daß** der Kopf (202) eine Hülse (212) aufweist, die in der Bohrung (204) angeordnet ist, um das orthopädische Schaftimplantat (100) vom Kopf (202) zu isolieren.

7. Navigiertes Schaftimplantat-Setzinstrument (150) nach Anspruch 6, **dadurch gekennzeichnet, daß** die Hülse (212) ein Polymer aufweist.

8. Navigiertes Schaftimplantat-Setzinstrument (150) nach Anspruch 6 oder 7, das ferner eine Halteplatte (214) aufweist, die über der Hülse (212) angeordnet ist, um die Hülse (212) in der Bohrung (204) zu halten.

9. Navigiertes Schaftimplantat-Setzinstrument (150) nach einem der vorhergehenden Ansprüche, **dadurch gekennzeichnet, daß** das Schaftverriegelungselement (300) und das Betätigungselement (400) getrennte längliche Elemente sind, die koaxial miteinander ausgerichtet sind, wobei das Setzinstrument (150) ferner eine Feder (310) aufweist, die angrenzend an das Schaftverriegelungselement (300) angebracht ist, um das Schaftverriegelungselement (300) zum Betätigungselement (400) vorzuspannen.

10. Navigiertes Schaftimplantat-Setzinstrument (150) nach einem der vorhergehenden Ansprüche, **dadurch gekennzeichnet, daß** der Verriegelungsringanschlagabschnitt (412) einen sich radial erstreckenden Flansch aufweist.

11. Navigiertes Schaftimplantat-Setzinstrument (150) nach einem der vorhergehenden Ansprüche, das ferner einen Hilfshandgriff (500) aufweist, der quer zur Schaftverriegelungsachse (254) angebracht ist.

12. Navigiertes Schaftimplantat-Setzinstrument (150) nach Anspruch 11, das ferner mehrere Handgriffbefestigungsformstücke (364, 366) aufweist, so daß der Handgriff (500) in mehreren Positionen quer zur Betätigungselementachse angebracht werden kann.

13. Navigiertes Schaftimplantat-Setzinstrument (150) nach einem der vorhergehenden Ansprüche, **dadurch gekennzeichnet, daß** das Setzinstrument (150) mehrere Bezugselementbefestigungsformstücke (372, 374) aufweist, so daß das Bezugselement (550) in mehreren Positionen angebracht werden kann.

## Revendications

1. Dispositif d'insertion navigué d'un implant à tige (150) pour une utilisation avec un implant orthopédique à tige (100) et un système de navigation chirurgical durant une chirurgie de remplacement articulaire sur un os, le dispositif d'insertion (150) comprenant :
un organe d'engagement de tige (200) pouvant être engagé avec l'implant orthopédique à tige (100) dans un agencement relatif rigide et incluant une tête en forme d'anneau (202) ayant un alésage (204) avec un axe d'alésage pour recevoir un col (104) de l'implant orthopédique à tige (100);
un organe de référence (550) pouvant être suivi par le système de navigation chirurgical, l'organe de référence (550) étant soutenu dans une relation rigide connue à l'implant orthopédique à tige (100) de telle sorte que le système de navigation chirurgical puisse déterminer la position et l'orientation de l'implant orthopédique à tige (100) par rapport à l'os par la détection de la position et de l'orientation de l'organe de référence (550) et la résolution de la position et de l'orientation de l'implant orthopédique à tige (100) d'après une relation connue entre l'organe de référence (550) et l'implant orthopédique à tige (100);
un organe de verrouillage de tige (300) monté au dispositif d'insertion pour une translation le long d'un axe de verrouillage de tige (254) transversal à l'axe d'alésage de la tête (202), l'organe de verrouillage de tige (300) étant mobile entre une première position dans laquelle le col (104) de l'implant orthopédique à tige (100) peut être dégagé de la tête (202) et une deuxième position dans laquelle le col (104) ne peut pas être dégagé de la tête (202) ; et
un actionneur (400) monté au dispositif d'insertion (150) pour une rotation et une translation le long de l'axe de verrouillage de tige (254), l'actionneur (400) étant capable de convertir une entrée en rotation à l'actionneur (400) en un mouvement linéaire pour déplacer l'organe de verrouillage de tige (300) de la première position à la deuxième position, où l'actionneur (400) inclut une portion de butée de l'anneau de verrouillage (412) et le dispositif d'insertion d'un implant à tige (150) comprend en plus un anneau de verrouillage (386) vissé sur le dispositif d'insertion (150), l'anneau de verrouillage (386) étant mobile entre une première position dans laquelle il est espacé de la portion de butée de l'anneau de verrouillage (412) et une deuxième position dans laquelle il vient en butée contre la portion de butée de l'anneau de verrouillage (412).

2. Dispositif d'insertion navigué d'un implant à tige (150) de la revendication 1 **caractérisé en ce que** l'organe de référence (550) est détectable en utilisant une technologie de détection sélectionnée parmi le groupe consistant en une technologie de type électromagnétique, acoustique, et d'imagerie.

3. Dispositif d'insertion navigué d'un implant à tige (150) de la revendication 2 **caractérisé en ce que** l'organe de référence (550) produit activement un signal détectable par le système de navigation chirurgical.

4. Dispositif d'insertion navigué d'un implant à tige (150) de la revendication 2 **caractérisé en ce que** l'organe de référence (550) est détecté passivement par le système de navigation chirurgical.

5. Dispositif d'insertion navigué d'un implant à tige (150) de l'une quelconque des revendications précédentes **caractérisé en ce que** le système de navigation chirurgical est capable de résoudre et d'afficher un ou plusieurs paramètres de position de l'implant orthopédique à tige (100) par rapport à l'os sélectionné(s) à partir de la liste consistant en une profondeur, une rotation, une inclinaison antérieure-postérieure, et une inclinaison médiale-latérale.

6. Dispositif d'insertion navigué d'un implant à tige (150) de l'une quelconque des revendications précédentes **caractérisé en ce que** la tête (202) inclut un manchon (212) disposé dans l'alésage (204) pour isoler l'implant orthopédique à tige (100) de la tête (202).

7. Dispositif d'insertion navigué d'un implant à tige (150) de la revendication 6 **caractérisé en ce que** le manchon (212) comprend un polymère.

8. Dispositif d'insertion navigué d'un implant à tige (150) de la revendication 6 ou 7 comprenant en plus une plaque de retenue (214) disposée sur le manchon (212) pour retenir le manchon (212) dans l'alésage (204).

9. Dispositif d'insertion navigué d'un implant à tige (150) de l'une quelconque des revendications précédentes **caractérisé en ce que** l'organe de verrouillage de tige (300) et l'actionneur (400) sont des organes allongés séparés alignés coaxialement l'un avec l'autre, le dispositif d'insertion (150) comprenant en plus un ressort (310) monté de manière adjacente à l'organe de verrouillage de tige (300) pour maintenir l'organe de verrouillage de tige (300) vers l'actionneur (400).

10. Dispositif d'insertion navigué d'un implant à tige (150) de l'une quelconque des revendications précédentes **caractérisé en ce que** la portion de butée de l'anneau de verrouillage (412) comprend une bride s'étendant radialement.

11. Dispositif d'insertion navigué d'un implant à tige (150) de l'une quelconque des revendications précédentes comprenant en plus une poignée auxiliaire (500) montée de manière transversale à l'axe de verrouillage de tige (254).

12. Dispositif d'insertion navigué d'un implant à tige (150) de la revendication 11 comprenant en plus une pluralité d'accessoires de montage de poignée (364, 366) de telle sorte que la poignée (500) puisse être montée dans une pluralité de positions transversales à l'axe de l'actionneur.

13. Dispositif d'insertion navigué d'un implant à tige (150) de l'une quelconque des revendications précédentes **caractérisé en ce que** le dispositif d'insertion (150) inclut une pluralité d'accessoires de montage de l'organe de référence (372, 374) de telle sorte que l'organe de référence (550) puisse être monté dans une pluralité de positions.
